(19) Europäisches Patentamt / European Patent Office / Office européen des brevets

(11) EP 1 797 879 A1

(12) EUROPEAN PATENT APPLICATION
published in accordance with Art. 158(3) EPC

(43) Date of publication:
20.06.2007 Bulletin 2007/25

(21) Application number: 05782332.0

(22) Date of filing: 09.09.2005

(51) Int Cl.:
*A61K 31/427* (2006.01) *A61K 31/407* (2006.01)
*A61P 31/04* (2006.01) *C07D 477/00* (2006.01)

(86) International application number:
PCT/JP2005/016611

(87) International publication number:
WO 2006/040893 (20.04.2006 Gazette 2006/16)

(84) Designated Contracting States:
AT BE BG CH CY CZ DE DK EE ES FI FR GB GR HU IE IS IT LI LT LU LV MC NL PL PT RO SE SI SK TR

(30) Priority: 08.10.2004 JP 2004296349

(71) Applicant: Dainippon Sumitomo Pharma Co., Ltd.
Osaka 541-8524 (JP)

(72) Inventors:
- SUNAGAWA, Makoto
Itami-shi, Hyogo 664-0875 (JP)
- UEDA, Yutaka,
c/o Dainippon Sumitomo Pharma
Osaka-shi, Osaka 5540022 (JP)
- KANAZAWA, Katsunori,
c/o Dainippon Sumitomo Pharma
Osaka-shi, Osaka 5540022 (JP)

(74) Representative: Vossius & Partner
Siebertstrasse 4
81675 München (DE)

(54) **NOVEL ANTIMICROBIAL MEDICINE**

(57) An antimicrobial combination medicament for injection comprising a β-lactam compound represented by the formula [1],

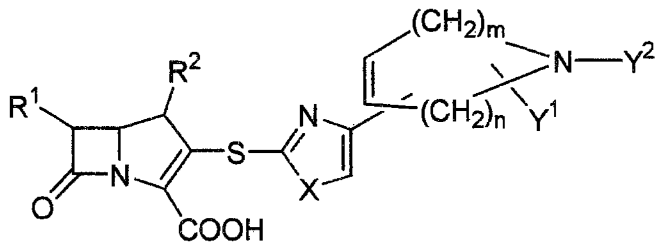

[1]

wherein $R^1$ is lower alkyl or the like, $R^2$ is H or lower alkyl group, X is O, S or NH; m and n are 0 to 4, provided that the sum of m and n is 0 to 4; $Y^1$ is halogen or the like; and $Y^2$ is hydrogen, optionally substituted lower alkyl group or the like; and carbapenems such as meropenem.

EP 1 797 879 A1

**Description**

TECHNICAL FIELD

The present invention relates to an antimicrobial medicament containing a β-lactam compound represented by the formula [1] below in combination with a carbapenem.

BACKGROUND ART

By the wide clinical application of the third-generation cephalosporins, Gram-positive bacteria have become to be frequently isolated. Particularly, methicillin-registant Staphylococcus aureus (hereinafter, abbreviated as MRSA) has been more frequently isolated, and becomes a serious problem in clinical field, because infectious diseases caused by MRSA are difficult to be treated. Although vancomycin has been broadly used for infectious diseases caused by MRSA in these days, it has a defect in difficulty of administration because of its side effects, and further glycopeptide-resistant bacteria are supposed to increase in future by administration thereof.

Moreover, it has recently been reported increase in isolation of methicillin-resistant and coagulase-negative Staphylococci (MRCNS). Under these circumstances, it has been desired to develop a safer medicament having excellent anti-MRSA and MRCNS activities.

The present inventors had intensively studied, and found that a compound [1] below shows excellent antibacterial activities against Gram-positive bacteria, especially MRSA and MRCNS and filed a patent application (See WO 02/38564).

DISCLOSURE OF INVENTION

The present inventors have further studied, and found that by concomitant administration of a compound [1] below and a carbapenem, not only mutual supplement of each spectrum, but also enhancement of antibacterial activities of the carbapenem is attained and shows superior antibacterial activities than ones of known antibacterial agents. Thus the present invention has been completed.

The present invention relates to an antimicrobial medicament containing a combination of a β-lactam compound [1] below and a carbapenem.

Namely, the present invention relates to an antimicrobial medicament containing a combination of a β-lactam compound represented by the following formula,

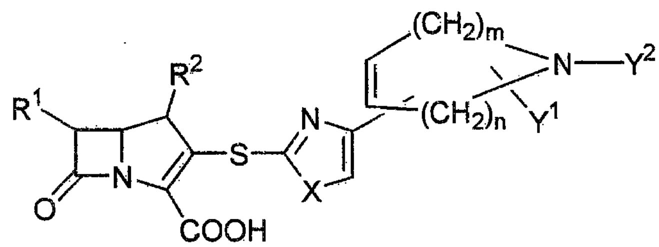

[1]

wherein $R^1$ is lower alkyl group or lower alkyl group substituted by hydroxy group, $R^2$ is hydrogen atom or lower alkyl group, X is O, S or NH, m and n are independently 0 to 4 and the sum of m and n is 0 to 4, $Y^1$ is halogen atom, cyano group, hydroxy group optionally protected, amino group optionally protected, lower alkyloxy group, lower alkylamino group, carboxyl group optionally protected, carbamoyl group optionally substituted or lower alkyl group optionally substituted, and $Y^2$ is hydrogen atom, lower alkyl group optionally substituted, cyano group or -C($R^3$)=N$R^4$ (wherein $R^3$ and $R^4$ are independently hydrogen atom, amino group optionally protected or substituted, or lower alkyl group optionally substituted, or $R^3$ and $R^4$ may combine with the carbon atom and nitrogen atom to which they bond to form a 5 to 7 membered heterocyclic ring which may be substituted), provided that 1 to 4 $Y^1$s may present on the same ring and 2 $Y^1$s may present on the same carbon atom,
its pharmaceutically acceptable salt or its nontoxic ester; and a carbapenem.

The present invention relates to the above antimicrobial medicament, wherein in the formula [1] X is S, and the sum of m and n is 2 or 3.

The present invention relates to the above antimicrobial medicament, wherein in the formula [1] $R^1$ is 1-(R)-hydroxyethyl.

The present invention relates to the above antimicrobial medicament, wherein in the formula [1] $R^1$ is 1-(R)-hy-

droxyethyl, $R^2$ is methyl, X is S, the sum of m and n is 2 or 3, $Y^1$ is methyl or hydroxymethyl, and $Y^2$ is hydrogen atom.

The present invention relates to the above antimicrobial medicament, wherein the β-lactam compound [1] is (4R, 5S, 6S)-6-[(1R)-1-hydroxyethyl]-4-methyl-3-({4-[(5S)-5-methyl-2, 5-dihydro-1H-pyrrol-3-yl]-1, 3-thiazol-2-yl}sulfanyl)-7-oxo-1-azabicyclo[3.2.0]hept-2-ene-2-carboxylic acid.

The present invention relates to the above antimicrobial medicament, wherein the β-lactam compound [1] is (4R, 5S, 6S)-6-[(1R)-1-hydroxyethyl]-3-({4-[(5R)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid, or (4R,5S,6S)-6-[(1R)-1-hydroxyethyl]-3-({4-[(5S)-5-(hydroxymethyl)-2, 5-dihydro-1H-pyrrol-3-yl]-1, 3-thiazol-2-yl}thio)-4-methyl-7-oxo-1-azabicyclo[3.2.0]hept-2-ene-2-carboxylic acid.

The present invention relates to the above antimicrobial medicament, wherein the carbapenem is meropenem, imipenem, panipenem, biapenem, ertapenem, doripenem (S-4661), CS-023 or ME-1036.

The present invention relates to the above antimicrobial medicament containing combination of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or its pharmaceutically acceptable salt; and meropenem, imipenem or panipenem.

The present invention relates to the above antimicrobial medicament containing combination of (4R,5S,6S)-6-[(1R)-1-hydroxyethyl]-4-methyl-3-({4-[(5S)-5-methyl-2,5-dihydro-1H-pyrrol-3-yl]-1, 3-thiazol-2-yl}sulfanyl)-7-oxo-1-azabicyclo[3.2.0]hept-2-ene-2-carboxylic acid, or its pharmaceutically acceptable salt; and meropenem.

The present invention relates to the above antimicrobial medicament which is in the form of an injection or a vial or a kit.

The present invention relates to a method for treating infectious diseases comprising administering a β-lactam compound [1] or its pharmaceutically acceptable salt; and a carbapenem in the effective amount to a patient who needs it.

The present invention relates to a method for treating infectious diseases comprising administering combination of a β-lactam compound [1] or its pharmaceutically acceptable salt; and a carbapenem in the effective amount to a patient who needs it.

The present invention relates to use of combination of a β-lactam compound [1] or its pharmaceutically acceptable salt; and a carbapenem in the preparation of an antimicrobial medicament for treating infectious diseases.

The present invention relates to use of a β-lactam compound [1] or its pharmaceutically acceptable salt for combination of a carbapenem in the preparation of an antimicrobial medicament for treating infectious diseases.

The lower alkyl group used in the present invention includes a straight chain or branched chain $C_{1-6}$ alkyl group, for example, methyl, ethyl, n-propyl, isopropyl, n-butyl, isobutyl, tert-butyl, n-pentyl and n-hexyl. The lower alkyl group substituted by a hydroxy group includes ones having 1 to 6 carbon atoms, for example, hydroxymethyl, 1-hydroxyethyl, 2-hydroxyethyl, 1-hydroxy-1-methylethyl, 1-hydroxypropyl, and 2-hydroxypropyl. The lower alkoxy group includes a straight chain or branched chain $C_{1-6}$ alkyloxy group, for example methoxy, ethoxy, n-propoxy, isopropoxy, n-butoxy, isobutoxy, tert-butoxy, n-pentoxy and n-hexoxy. The lower alkylamino group includes amino group mono or di-substituted by a straight chain or branched chain $C_{1-6}$ alkyl group, for example methylamino, ethylamino, n-propylamino, isopropylamino, n-butylamino, isobutylamino, tert-butylamino, n-pentylamino, n-hexylamino, methyl ethylamino, dimethylamino, diethylamino, di(n-propyl)amino, di(isopropyl)amino, di(n-butyl)amino, di(n-pentyl)amino and di(n-hexyl)amino. The halogen atom includes fluorine atom, chlorine atom, bromine atom and iodine atom. The 5 to 7 membered hetero cyclic ring includes, for example 3,4-dihydro-2-H-pyrrole ring, 2,3,4,5-tetrahydropyridine ring, 3,4,5,6-tetrahydro-2-H-azepine ring, etc. The substituent of lower alkyl group optionally substituted includes a hydroxy group, a lower alkoxy group, a lower alkylthio group, a lower alkylsulfinyl group, a lower alkylsulfonyl group, a lower alkylcarbonyl group, a lower alkylcarbonyloxy group, a lower alkoxycarbonyl group, a carboxyl group, a halogen atom, cyano group, $-NR^6R^7$ (wherein $R^6$ and $R^7$ are independently hydrogen atom or a lower alkyl group, or $R^6$ and $R^7$ may be combined together with the nitrogen atom to form a 5-7 membered ring such as pyrrolidine, piperidine, azepane, morpholine, piperazine, or a N-lower alkyl piperazine), $-CONR^6R^7$ (wherein $R^6$ and $R^7$ are the same as defined above), $-NR^{6a}COR^{7a}$ (wherein $R^{6a}$ and $R^{7a}$ are independently hydrogen atom or a lower alkyl group), $-OCONR^6R^7$ (wherein $R^6$ and $R^7$ are the same as defined above), $-SO_2NR^6R^7$ (wherein $R^6$ and $R^7$ are the same as defined above), $-NR^{6a}SO_2NR^6R^7$ (wherein $R^{6a}$, $R^6$ and $R^7$ are the same as defined above), $-NR^{6a}CONR^6R^7$ (wherein $R^{6a}$, $R^6$ and $R^7$ are the same as defined above), and $-COOCH_2OCOR^8$ (wherein $R^8$ is a lower alkyl group). These substituents may be protected by a suitable protective group. The substituted positions are not limited as far as chemically possible, but one or more substituents are possible.

The lower alkylcarbonyl group includes a straight chain or branched chain $C_{2-7}$ alkylcarbonyl group, for example methylcarbonyl, ethylcarbonyl, n-propylcarbonyl, isopropylcarbonyl, n-butylcarbonyl, isobutylcarbonyl, tert-butylcarbohyl, n-pentylcarbonyl and n-hexylcarbonyl. The lower alkylcarbonyloxy group includes a straight chain or branched chain $C_{2-7}$ alkylcarbonyloxy group, for example methylcarbonyloxy, ethylcarbonyloxy, n-propylcarbonyloxy, isopropylcarbonyloxy, n-butylcarbonyloxy, isobutylcarbonyloxy, tert-butylcarbonyloxy, n-pentylcarbonyloxy and n-hexylcarbonyloxy.

The lower alkoxycarbonyl group includes a straight chain or branched chain $C_{2-7}$ alkoxycarbonyl group, for example methoxycarbonyl, ethoxycarbonyl, n-propoxycarbonyl, isopropoxycarbonyl, n-butoxycarbonyl, isobutoxycarbonyl, tert-butoxycarbonyl, n-pentoxycarbonyl and n-hexoxycarbonyl. The lower alkyl portion of lower alkylthio group, lower alkylsulfinyl group and lower alkylsulfonyl group includes a straight chain or branched chain $C_{1-6}$ alkyl group, for example, methyl, ethyl, n-propyl, isopropyl, n-butyl, isobutyl, tert-butyl, n-pentyl and n-hexyl. The substituent of carbamoyl group optionally substituted includes one or two lower alkyl groups, and the carbamoyl group substituted includes pyrrolidine, piperidine and azepane, which is formed with the nitrogen atom of the carbamoyl group. The substituent of the amino group optionally substituted includes one or two lower alkyl groups, and the amino group substituted includes pyrrolidine, piperidine, and azepane, which is formed with the nitrogen atom of the amino group.

The substituent of the 5 to 7 membered heterocyclic group optionally substituted includes, for example a lower alkyl group, a hydroxy group, a lower alkoxy group, a lower alkylcarbonyl group, a lower alkylcarbonyloxy group, a lower alkyloxycarbonyl group, a carboxyl group, a halogen atom, and cyano group.

The protecting group for carboxyl group may be any conventional protecting groups, but preferably for example, a straight chain or branched chain lower alkyl group having 1 to 5 carbon atoms (e.g., methyl, ethyl, isopropyl, tert-butyl, etc.), a halogeno $C_{1-5}$ lower alkyl group (e.g., 2-iodoethyl, 2,2,2-trichloroethyl, etc.), an $C_{1-5}$ alkoxymethyl group (e.g., methoxymethyl, ethoxymethyl, isobutoxymethyl, etc.), a $C_{1-5}$ lower aliphatic acyloxymethyl group (e.g., acetoxymethyl, propionyloxymethyl, butyryloxymethyl, pivaloyloxymethyl, etc.), a 1-($C_{1-5}$) lower alkoxycarbonyloxyethyl group (e.g., 1-ethoxycarbonyloxyethyl), a substituted or unsubstituted aralkyl group (e.g., benzyl, p-methoxybenzyl, o-nitrobenzyl, p-nitrobenzyl), a $C_{3-7}$ lower alkenyl group (e.g., allyl, 3-methylallyl), benzhydryl group, or phthalidyl group.

The protecting group for a hydroxy group or an amino group may be any conventional one, and preferably a $C_{1-5}$ lower alkoxycarbonyl group (e.g., tert-butyloxycarbonyl), a halogeno $C_{1-5}$ alkoxycarbonyl group (e.g., 2-iodoethyloxycarbonyl, 2,2,2-trichloroethyloxycarbonyl), a substituted or unsubstituted $C_{3-7}$ lower alkenyloxycarbonyl group (e.g., allyloxycarbonyl), a substituted or unsubstituted aralkyloxycarbonyl group (e.g., benzyloxycarbonyl, p-methoxybenzyloxycarbonyl, o-nitro benzyloxycarbonyl, p-nitrobenzyloxycarbonyl), or a trialkylsilyl group (e.g., trimethylsilyl, triethylsilyl, tert-butyldimethylsilyl).

The preferable substituents of $Y^1$ on the β-lactam compound of the formula [1] are a $C_{1-3}$ alkyl group such as methyl, ethyl, isopropyl, etc., hydroxymethyl, chloromethyl, fluoromethyl, methoxymethyl, carbamoyloxymethyl ($-CH_2OCONH_2$), ureidomethyl ($-CH_2NHCONH_2$), sulfamoylmethyl ($-CH_2SO_2NH_2$), sulfamoylaminomethyl ($-CH_2NHSO_2NH_2$), carbamoyl, etc., and preferable substituents of $Y^2$ are hydrogen atom, a $C_{1-3}$ alkyl group such as methyl, ethyl, isopropyl, etc., iminomethyl (-CH=NH), $-C(CH_3)=NH$, etc., preferable $R^2$ is methyl, etc., and preferable $R^1$ is 1-(R)-hydroxyethyl.

The pharmaceutically acceptable salt of the compound of the above formula [1] is a conventional non-toxic salt. Such salts include, as a salt with an intramolecular carboxylic acid, a salt with an inorganic base such as sodium, potassium, calcium, magnesium, ammonium, a salt with an organic base such as triethylammonium, pyridinium, diisopropylammonium, or an intramolecular salt being formed with a cation at the 3-position side chain such as a quaternary ammonium ion. As a salt with an intramolecular base, a salt with an inorganic acid such as hydrochloric acid, sulfuric acid, phosphoric acid, or a salt with an organic acid such as formic acid, acetic acid, oxalic acid, methanesulfonic acid, or benzenesulfonic acid can be exemplified.

The non-toxic ester of the compound of the formula [1] includes a conventional pharmaceutically acceptable ester at the 2-carboxyl group of carbapenem antibacterial agents, and may be esters being able to be easily hydrolyzed in a living body, for example esters with acetoxymethyl, pivaloyloxymethyl, 1-(ethoxycarbonyloxy)ethyl, and phthalidyl.

The β-lactam compound of the formula [1], or a pharmaceutically acceptable salt thereof, or a non-toxic ester thereof may be in the form of an anhydride thereof, a hydrate thereof, or a solvate thereof.

The compound of the formula [1] may have optical isomers based on the asymmetric carbon atoms at the 4-, 5- and 6-positions of the carbapenem nucleus, as shown in the following formula,

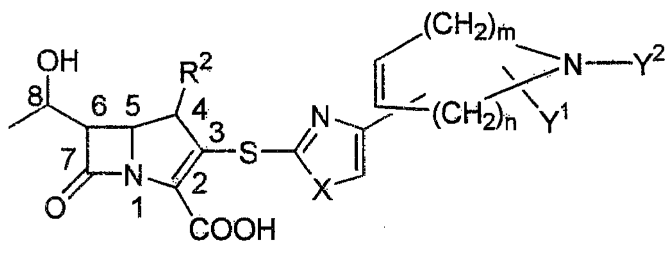

and these isomers are all conveniently expressed by only one formula. However, the scope of the present invention should not be construed to be limited thereby, and includes all isomers and a mixture of isomers based on each asymmetric carbon atom. As an isomer due to substiuent $Y^1$ may exist, the scope of the present invention includes all isomers and a mixture of the isomers. There are illustrated compounds in which the 4-position carbon atom has an R-eonfiguration

and the 5-position carbon atom has an S-configuration, such as (4R,5S,6S)-compounds or (4R,5S,6R)-compounds, when $R^2$ is a lower alkyl group. Moreover, when $R^1$ is 1-hydroxyethyl group, the compound [1] may have isomers having an R-configuration or an S-configuration at the 8-position, as shown in the above formula, the preferable one is ones having an R-configuration at the 8-position.

The β-lactam compound of the formula [1], a pharmaceutically acceptable salt thereof, or a non-toxic ester thereof are known, and can be prepared in accordance of the methods described in WO 02/38564.

The preferable compounds among β-lactam compounds of the formula [1] are illustrated below.

(4R,5S,6S)-6-[(1R)-1-Hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid(abbreviated as compound 1).
(4R,5S,6S)-6-[(1R)-1-Hydroxyethyl]-4-methyl-3-({4-[(5R)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid.
(4R,5S,6S)-6-[(1R)-1-Hydroxyethyl]-4-methyl-3-({4-[(6S)-6-methyl-1,2,3,6-tetrahydro-4-pyridinyl]-1,3-thiazol-2-yl}sulfanyl)-7-oxo-1-azabicyclo[3.2.0]hept-2-ene-2-carboxylic acid.
(4R,5S,6S)-6-[(1R)-1-Hydroxyethyl]-4-methyl-3-({4-[(6R)-6-methyl-1,2,3,6-tetrahydro-4-pyridinyl]-1,3-thiazol-2-yl}sulfanyl)-7-oxo-1-azabicyclo[3.2.0]hept-2-ene-2-carboxylic acid.
(4R,5S,6S)-6-[(1R)-1-Hydroxyethyl]-3-({4-[(2R)-2-(hydroxymethyl)-1,2,3,6-tetrahydro-4-pyridinyl]-1,3-thiazol-2-yl}sulfanyl)-4-methyl-7-oxo-1-azabicyclo[3.2.0]hept-2-ene-2-carboxylic acid.
(4R,5S,6S)-6-[(1R)-1-Hydroxyethyl]-3-({4-[(2S)-2-(hydroxymethyl)-1,2,3,6-tetrahydro-4-pyridinyl]-1,3-thiazol-2-yl}sulfanyl)-4-methyl-7-oxo-1-azabicyclo[3.2.0]hept-2-ene-2-carboxylic acid.
(4R,5S,6S)-6-[(1R)-1-Hydroxyethyl]-4-methyl-3-({4-[(2R)-2-methyl-1,2,3,6-tetrahydro-4-pyridinyl]-1,3-thiazol-2-yl}sulfanyl)-4-methyl-7-oxo-1-azabicyclo[3.2.0]hept-2-ene-2-carboxylic acid.
(4R,5S,6S)-6-[(1R)-1-Hydroxyethyl]-4-methyl-3-({4-[(2S)-2-methyl-1,2,3,6-tetrahydro-4-pyridinyl]-1,3-thiazol-2-yl}sulfanyl)-4-methyl-7-oxo-1-azabicyclo[3.2.0]hept-2-ene-2-carboxylic acid.
(4R,5S,6S)-6-[(1R)-1-Hydroxyethyl]-4-methyl-3-({4-[(2S)-2-methyl-2,5-dihydro-1H-pyrrol-3-yl]-1,3-thiazol-2-yl})sulfanyl)-7-oxo-1-azabicyclo[3.2.0]hept-2-ene-2-carboxylic acid.
(4R,5S,6S)-6-[(1R)-1-Hydroxyethyl]-4-methyl-3-({4-[(2R)-2-methyl-2,5-dihydro-1H-pyrrol-3-yl]-1,3-thiazol-2-yl}) sulfanyl)-7-oxo-1-azabicyclo[3.2.0]hept-2-ene-2-carboxylic acid.
(4R,5S,6S)-6-[(1R)-1-Hydroxyethyl]-3-({4-[(2S)-2-(hydroxymethyl)-2,5-dihydro-1H-pyrrol-3-yl]-1,3-thiazol-2-yl}sulfanyl)-4-methyl-7-oxo-1-azabicyclo[3.2.0]hept-2-ene-2-carboxylic acid.
(4R,5S,6S)-6-[(1R)-1-Hydroxyethyl]-3-({4-[(2R)-2-(hydroxymethyl)-2,5-dihydro-1H-pyrrol-3-yl]-1,3-thiazol-2-yl}) sulfanyl)-4-methyl-7-oxo-1-azabicyclo[3.2.0]hept-2-ene-2-carboxylic acid.
(4R, 5S, 6S)-6-[(1R)-1-Hydroxyethyl]-3-({4-[(2R)-2-(methoxymethyl)-2, 5-dihydro-1H-pyrrol-3-yl]-1,3-thiazol-2-yl}) sulfanyl)-4-methyl-7-oxo-1-azabicyclo[3.2.0]hept-2-ene-2-carboxylic acid.
(4R,5S,65)-6-[(1R)-1-Hydroxyethyl]-4-methyl-3-{[4-(6-methyl-1,2,5,6-tetrahydro-3-pyridinyl)-1,3-thiazol-2-yl]sulfanyl]-7-oxo-1-azabicyclo[3.2.0]hept-2-ene-2-carboxylic acid.
(4R,5S,6S)-6-[(1R)-1-Hydroxyethyl]-4-methyl-3-({4-[(6R)-6-methyl-1,2,5,6-tetrahydro-3-pyridinyl]-1,3-thiazol-2-yl}sulfanyl)-}-7-oxo-1-azabicyclo[3.2.0]hept-2-ene-2-carboxylic acid.
(4R,5S,6S)-6-[(1R)-1-Hydroxyethyl]-4-methyl-3-({4-[(6S)-6-methyl-1,2,5,6-tetrahydro-3-pyridinyl]-1,3-thiazol-2-yl}sulfanyl)-7-oxo-1-azabicyclo[3.2.0]hept-2-ene-2-carboxylic acid.
(4R,5S,6S)-6-[(1R)-1-Hydroxyethyl]-3-({4-[(6S)-6-(hydroxymethyl)-1,2,5,6-tetrahydro-3-pyridinyl]-1,3-thiazol-2-yl}sulfanyl)-4-methyl-7-oxo-1-azabicyclo[3.2.0]hept-2-ene-2-carboxylic acid.
(4R,5S,6S)-3-{(4-((2R)-2-{[(Aminocarbonyl)oxy]oxy]ethyl}-1,2,3,6-tetrahydro-4-pyridinyl)-1,3-thiazol-2-yl]sulfanyl}-6-[(1R)-1-hydroxyethyl]-4-methyl-7-oxo-1-azabicyclo[3.2.0]hept-2-ene-2-carboxylic acid.
(4R,5S,6S)-6-[(1R)-1-Hydroxyethyl]-3-({4-[(6R)-6-(hydroxymethyl)-1,2,5,6-tetrahydro-3-pyridinyl]-1,3-thiazol-2-yl}sulfanyl)-4-methyl-7-oxo-1-azabicyclo[3.2.0]hept-2-ene-2-carboxylic acid.
(4R,5S,6S)-6-[(1R)-1-Hydroxyethyl]-3-({4-[(5R)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid.
(4R,5S,6S)-6-[(1R)-1-Hydroxyethyl]-3-({4-[(5S)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid.

And a pharmaceutically acceptable salt of the above compound.

On the other hand, carbapenems used in this invention are not limited, and include meropenem, imipenem, panipenem, biapenem, ertapenem, doripenem (S-4661), CS-023 and ME-1036, preferably carbapenems which show excellent antibacterial activities against Gram negative bacteria including Pseudomonas aeruginosae, such as meropenem, imipenem, panipenem, biapenem, doripenem (S-4661), and CS-023, especially preferably meropenem.

Doripenem (S-4661) is described in the Journal of Antibiotics Vol. 49, No.2, 199-205, 1996 and Japanese Patent Publication A Hei 5-294970 and has a following formula:

Chiral

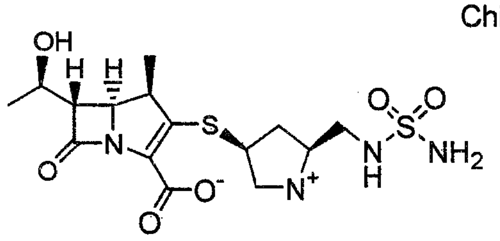

CS-023 is described in the Journal of Antibiotics Vol. 56, No. 6, 565-579, 2003 and has a following formula:

Chiral

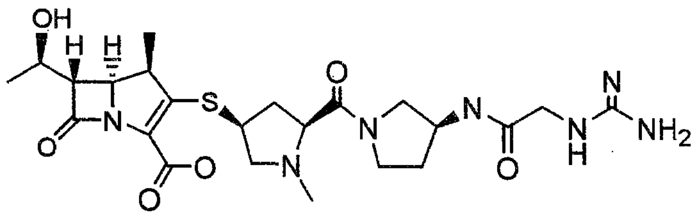

ME-1036 is described in Antimicrobial Agents and Chemotherapy, Aug. 2004, 2381-2837 and WO 02/42321 and has a following formula:

Chiral

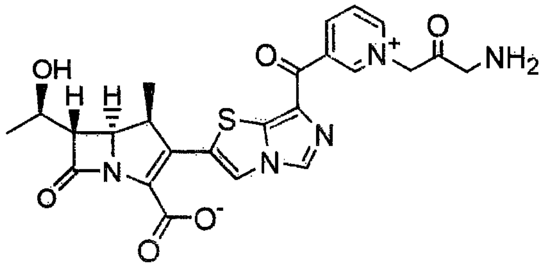

The ratio of a β-lactam compound [1] and a carbapenem is not limited and according to pharmacokinetic property of each drug the ratio should be changed. In case of administration in the form of a combination of both compounds, such ratio that the value of a β-lactam compound [1]/a carbapenem in a living body maintains preferably a range of 1/10 to 10 is preferable, especially in case of meropenem, such ratio that the value of a β-lactam compound [1]/meropenem maintains a range of 1 to 4 for possible long time is preferable.

The administration forms of the present concomitant medicament used for an antimicrobial agent for treating infectious diseases include parenteral administration such as an intravenous injection, an intramuscular injection, an infusion, an intrarectal administration, or inhalation. The medicament may contain other suitable medicines according to the disease of a patient.

The suitable administration forms as mentioned above may be prepared according to the conventinal method using active ingredients with a conventional pharmaceutically acceptable carriers, excipients, binders, or stabilizers.

For example, injections (e.g., subcutaneous injection, intravenous injection, intramuscular injection, intraperitoneal injection and so on)can be prepared by dissolving, suspending or emulsifying active ingredients with a dispersing agent (e.g., Tween 80 (AtlasPowder, USA), HCO 60 (Nikko Chemicals), polyethyleneglycol, carboxymethylcellulose, sodium alginate), preservatives (e.g., methylparaben, propylparaben, benzyl alcohol, chlorobutanol, phenol), tonicity agents (e.g., sodium chloride, glycerin, solbitol, glucose, invert sugar) and so on in an aqueous solvent (e.g., distilled water, saline solution, Ringer's solution) or an oily solvent (e.g., vegetable oil such as olive oil, sesame oil, cottonseed oil or corn oil, propylene glycol). If necessary, an additive such as a solubilizing agent (e.g., sodium salicylate, sodium acetate), a stabilizing agent (e.g., human serum albumin), or a soothing agent (e.g., benzalkonium chloride, procaine

hydrochloride) may be added thereto.

Regarding the antimicrobial medicament of the present invention comprising a combination of a β-lactam compound [1], its pharmaceutically acceptable salt or its non-toxic ester; and a carbapenem, both active ingredients can be pareneterally administered together or separately. In case that each active ingredient is separately formed in the preparation, each ingredient may be mixed with diluents on administration, and separately administered in intervals to the same subject.

The dosage of the antimicrobial medicament of the present invention varies according to the symptom, age, body weight, the administration form, the frequency of the administration, etc., but usually in the range of 100mg to 12g per day for an adult, which is administered once or divided into several dosage units, preferably for several days. The dosage may be increased or decreased, if necessary.

In addition the ratio of the drugs contained is selected according to subject, age, body weight, the symptom, the administration term, the administration form, the administration route, combination of drugs, etc. Especially, in case of combination of a β-lactam compound [1] of the present invention and a carbapenem, the ratio of a β-lactam compound [1], its pharmaceutically acceptable salt or its nontoxic ester per a carbapenem is 0.1 to 10 weight parts, preferably 0.3 to 1.5weight parts. Especially when the carbapenem is meropenem trihydrate, a β-lactam compound [1], its pharmaceutically acceptable salt or its nontoxic ester per meropen is 0.1 to 10 weight parts, preferably 0.3 to 1.5 weight parts, more preferably 0.5 to 1.0 weight parts.

Example

Preparation 1 (Solution for injection)

Compound 1 (250mg) and meropenem trihydrate (250mg) were dissolved in J.P. (Japanese Pharmacopeia) saline solution (100ml). The solution was sterilized by aseptic filtration and was filled into a vial to prepare the solution for injection.

Preparation 2 (Solution for injection)

Compound 1 (250mg) and meropenem trihydrate (250mg) were dissolved in J.P. saline solution (100ml). The solution was sterilized by aseptic filtration and was filled into an ampoule to prepare the solution for injection.

Preparation 3 (Lyophilized preparation)

Compound 1 (250mg) and meropenem trihydrate (250mg) were dissolved in distilled water (50ml). The solution was sterilized by aseptic filtration and was filled into a vial. The filled solution was lyophilized to prepare the lyophilized preparation.

Preparation 4 (Powder-preparation)

Compound 1 (250mg) in powders and meropenem trihydrate (250mg) in powders were filled into a vial to prepare the powder. Reference example

The following 2 compounds were synthesized according to the method described in Example 1 of WO 02/38564.

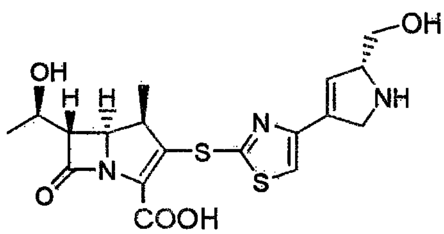

(4R,5S,6S)-6-[(1R)-1-Hydroxyethyl]-3-({4-[(5R)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid.

$^1$H NMR (300 MHz, $DCl_3$) δ 1.02 (3H, d, J=7.3 Hz), 1.20 (3H, d, J = 6.2 Hz), 3.19-3.30 (1H, m), 3.34 (1H, dd, J = 6.1, 2.7Hz), 3.84 (1H, dd, J = 12.6, 5.0 Hz), 3.95 (1H, dd, J = 12.6, 3.3 Hz), 4.13-4.22 (2H, in), 4.38-4.50 (2H, m), 6.27 (1H, s), 7.62 (1H, s).

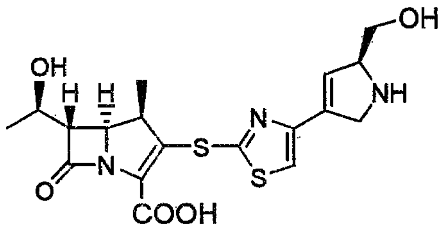

(4R,5S,6S)-6-[(1R)-1-Hydroxyethyl]-3-({4-[(5S)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid.
$^1$H NMR (300 MHz, $CDCl_3$) δ 0.93 (3H, d, J=7.3 Hz), 0.98 (3H, d, J = 6.4 Hz), 3.13-3.19 (1H, m), 3.33 (1H, dd, J = 6.1, 2.9Hz), 3.71 (1H, dd, J = 12.5, 5.1 Hz), 3.82 (1H, dd, J = 12.5, 3.4 Hz), 4.06-4.13 (2H, m), 4.28-4.33 (2H, m), 6.18 (1H, s), 7.51 (1H, s).

Test example Sensitivity test

1) Tested strains

The antibacterial activities were tested against various 18 bacteria (27 standard strains) which are representative bacteria caused clinically infectious disease (Staphylococcus aureus 3 strains, Staphylococcus epidermidis 1 strain, Micrococcus luteus 1 strain, Streptococcus pyogenes 1 strain, Enterococcus faecalis 1 strain, Enterococcus faecium 1 strain, Bacillus subtilis 1 strain, Escherichia coli 3 strains, Klebsiella pneumoniae 1 strain, Proteus mirabilis 1 strain, Proteus vulgaris 2 strains, Pseudomonas aeruginosa 3 strains, Serratia marcescens 2 strains, Enterobacter aerogenes 1 strain, Stenotrophomonas maltophilia 1 strain, Enterobacter cloacae 1 strain, Citrobacter freundii 1 strain, Moraxella catarrhalis 2 strains).

In addition, the antibacterial activities were tested against strains which are comparatively less sensitive to β-lactam compounds, namely clinically isolated 13 strains of Acinetobacter calcoaceticus and Acinetobacter lwoffi, clinically isolated 11 strains of Serratia marcescens, clinically isolated 12 strains of Burkholderia cepacia, and clinically isolated 15 strains of Pseudomonas aeruginosa.

2) Test method

According to the standard method of Japan Chemotherapeutic Society (Chemotherapy, vol.129, p76-79 (1981) ) the test was carried out using the agar dilution method. After dissolving test samples in distilled water, agar medium containing the test samples was prepared in such a manner that the final concentration became twice serial dilution by pouring serially diluted combination drug solution or a single drug solution into agar medium. As agar medium was used Mueller Hinton Agar medium (MHA). Then, each strain was cultivated over night at 37°C in an incubator and suspended in buffered saline solution with gelatin (BSG) in the concentration of about $10^6$CFU/ml. The suspension (about 5μl) containing test strains was inoculated to the medium containing the test samples by Micro Planter. After cultivating the agar medium at 37°C for about 20hours, growth of the strains was checked, and the minimum inhibitory concentration wherein the test sample inhibited the bacterial growth was determined (MIC, μg/ml).

3) Preparation of test samples

Each drug was weighed, and dissolved in distilled water to give a test sample. When the drugs are used in combination, two kinds of solution containing each drug were mixed so as to be various ratio of concentration to give a mixed solution. When the sample is subjected to sensitivity test, this mixed solution was diluted by twice serial dilution method.

Furthermore, when the drug is solely used, each test sample was used as it is, and diluted by twice serial dilution method from the maximum concentration to the minimum concentration.

4) Judgment of combination effect

When each concentration of the test drugs containing in a combination drug which shows MIC against the strain is less than 1/2 comparing with MIC of each drug, the effect of the combination drug in said ratio was judged to be additive or synergistic.

In each table, MIC values of carbapenems, other anibiotics, compound 1, and combination drug were indicated. FIC index based on MIC value was calculated. FIC index is generally used as a standard evaluation parameter of the combination effect in combination of antibiotics (e.g., The Japanese Journal of Antibiotics vol.58, p168-178, 2005). Namely, when FIC index calculated by the following formula is equal or less than 1, the enhanced effect of the combination drug in said ratio is considered as positive. When FIC index is over 1, the enhanced effect of the combination drug in said ratio is considered as negative.

Formula of FIC index: FIC index = (MIC of Drug A in combination/MIC of Drug A alone) + (MIC of Drug B in combination/MIC of Drug B alone)

N.C. in tables means being not calculated.

Test 1

The combination effect of meropenem and compound 1 against the above standard strains were shown in Table 1-a to Table 1-d. The test method and preparations of the drug were done in accordance with above methods.
As shown in Table 1-a to Table 1-d, the enhanced effect was recognized in higher frequency especially against Gram negative strains. Test 2
The combination effect of meropenem and compound 1 against the above clinically isolated strains were shown in Table 2-a and Table 2-b. The test method and preparations of the test sample were done in accordance with above methods.
As shown in Table 2-a and Table 2-b, the enhanced effect against the clinically isolated strains was recognized likewise against the standard strains.

Test 3

The combination effect of compound 1 and imipenem, panipenem, biapenem, doripenem, piperacillin, ceftazidime, aztreonam or ciprofloxacin against the above standard strains were shown in Table 3-a to Table 3-h. The test method and preparations of the drug were done in accordance with above methods.
As shown in Table 3-e to Table 3-g, even in the same β-lactam compounds, the combination of either piperacillin belonging to penicillins, ceftazidime belonging to cephalosporins, or aztreonam belonging to monobactams with compound 1 showed remarkably high FIC index against considerable strains, and decrease of efficacy by combination, namely antagonism was recognized. In addition, as shown in Table 3-h in case of ciprofloxacin belonging to new quinolone antibiotics, its combination effect was hardly recognized.
On the contrary, as shown in Table 3-a to Table 3-d, when imipenem, panipenem, biapenem or doripenem belonging to carbapenems was combined with compound 1, all combinations showed FIC index equal or less than 1 against many strains, and the combination effect was recognized likewise meropenem.
In the tables MEPM or ME means meropenem, SM means compound 1, IPM means imipenem, PAPM means panipenem, BIPM means biapenem, DRPM means doripenem, PIPC means piperacillin, CAZ means ceftazidime, AZT means aztreonam, and CPFX means ciprofloxacin, respectively.
Numbers in each table mean the minimum inhibitory concentration (MIC) (μg/ml) in which each drug or combination drug inhibited growth of the strains.

Table 1-a

| | Strain No. | MEPM alone | SM alone | Concomitant drug (MEPM:SM=2:1) | | FIC index |
|---|---|---|---|---|---|---|
| | | | | MEPM | SM | |
| Gram positive bacteria | *S.aureus* 209P | 0.063 | ≦0.008 | ≦0.016 | ≦0.008 | N.C. |
| | *S.aureus* MS94088 (hetero-MRSA) | 4 | 0.25 | 0.5 | 0.25 | 1.1 |
| | *S.aureus* SP-7928 (homo-MRSA) | 32 | 1 | 2 | 1 | 1.1 |
| | *S.epidermidis* IAM1296 | 1 | 0.016 | 0.063 | 0.031 | 2.1 |
| | *M.luteus* ATCC9341 | 0.125 | ≦0.008 | ≦0.016 | ≦0.008 | N.C. |
| | *Str.pyogenes* Cook | ≦0.016 | ≦0.008 | ≦0.016 | ≦0.008 | N.C. |
| | *E.faecalis* ATCC19433 | 4 | 1 | 1 | 0.5 | 0.8 |
| | *E.faecium* ATCC19434 | 8 | 0.25 | 0.5 | 0.25 | 1.1 |
| | *B.subtilis* ATCC6633 | 0.063 | ≦0.008 | ≦0.016 | ≦0.008 | N.C. |
| Gram negative bacteria | *E.coli* NIHJ JC-2 | ≦0.016 | 0.125 | ≦0.016 | ≦0.008 | N.C. |
| | *E.coli* ML1410 | 0.031 | 0.25 | ≦0.016 | ≦0.008 | ≦0.5 |
| | *E.coli* ML1410 RP4 | 0.031 | 0.5 | ≦0.016 | ≦0.008 | ≦0.5 |
| | *K.pneumoniae* ATCC10031 | ≦0.016 | 0.031 | ≦0.016 | ≦0.008 | N.C. |
| | *P.mirabiris* GN2425 | 0.063 | 0.125 | 0.031 | 0.016 | 0.6 |
| | *P.vurgalis* OX-19 | 0.063 | 0.063 | 0.031 | 0.016 | 0.7 |
| | *P.vulgaris* GN7919 | 0.063 | 1 | 0.031 | 0.016 | 0.5 |
| | *S.marcescens* χ100 | 0.031 | 0.5 | 0.031 | 0.016 | 1.03 |
| | *S.marcescens* GN6473 | 0.063 | 4 | 0.031 | 0.016 | 0.5 |
| | *E.aerogenes* ATCC13048 | 0.063 | 8 | 0.031 | 0.016 | 0.5 |
| | *E.cloacae* GN7471 | 0.063 | 8 | 0.031 | 0.016 | 0.5 |
| | *C.freundii* GN346 | 0.063 | 8 | 0.031 | 0.016 | 0.5 |
| | *P.aeruginosa* IFO3451 | 0.5 | 8 | 0.5 | 0.25 | 1.03 |
| | *P.aeruginosa* TL-2666 | 1 | 16 | 1 | 0.5 | 1.03 |
| | *P.aeruginosa* TL-2667 | 4 | 16 | 4 | 2 | 1.1 |
| | *S.maltophilia* IID1275 | >32 | >128 | >32 | >16 | N.C. |
| | *M.catarrhalis* ATCC25238 | ≦0.016 | ≦0.008 | ≦0.016 | ≦0.008 | N.C. |
| | *M.catarrhalis* ATCC8176 | ≦0.016 | ≦0.008 | ≦0.016 | ≦0.008 | N.C. |

**Table 1-b**

| | Strain No. | MEPM alone | SM alone | Concomitant (MEPM:SM=1:1) | | FIC index |
|---|---|---|---|---|---|---|
| | | | | MEPM | SM | |
| Gram positive bacteria | *S.aureus* 209P | 0.063 | ≦0.008 | ≦0.016 | ≦0.016 | N.C. |
| | *S.aureus* MS94088 (hetero-MRSA) | 4 | 0.25 | 0.25 | 0.25 | 1.1 |
| | *S.aureus* SP-7928 (homo-MRSA) | 32 | 1 | 1 | 1 | 1.03 |
| | *S.epidermidis* IAM1296 | 1 | 0.016 | ≦0.016 | ≦0.016 | ≦1.02 |
| | *M.luteus* ATCC9341 | 0.125 | ≦0.008 | ≦0.016 | ≦0.016 | N.C. |
| | *Str.pyogenes* Cook | ≦0.016 | ≦0.008 | ≦0.016 | ≦0.016 | N.C. |
| | *E.faecalis* ATCC19433 | 4 | 1 | 1 | 1 | 1.3 |
| | *E.faecium* ATCC19434 | 8 | 0.25 | 0.125 | 0.125 | 0.5 |
| | *B.subtilis* ATCC6633 | 0.063 | ≦0.008 | ≦0.016 | ≦0.016 | N.C. |
| Gram negative bacteria | *E.coli* NIHJ JC-2 | ≦0.016 | 0.125 | ≦0.016 | ≦0.016 | N.C. |
| | *E.coli* ML1410 | 0.031 | 0.25 | ≦0.016 | ≦0.016 | ≦0.6 |
| | *E.coli* ML1410 RP4 | 0.031 | 0.5 | ≦0.016 | ≦0.016 | ≦0.5 |
| | *K.pneumoniae* ATCC10031 | ≦0.016 | 0.031 | ≦0.016 | ≦0.016 | N.C. |
| | *P.mirabiris* GN2425 | 0.063 | 0.125 | ≦0.016 | ≦0.016 | ≦0.4 |
| | *P.vurgalis* OX-19 | 0.063 | 0.063 | ≦0.016 | ≦0.016 | ≦0.5 |
| | *P.vulgaris* GN7919 | 0.063 | 1 | 0.031 | 0.031 | 0.5 |
| | *S.marcescens* χ 100 | 0.031 | 0.5 | ≦0.016 | ≦0.016 | ≦0.5 |
| | *S.marcescens* GN6473 | 0.063 | 4 | 0.031 | 0.031 | 0.5 |
| | *E.aerogenes* ATCC13048 | 0.063 | 8 | ≦0.016 | ≦0.016 | ≦0.3 |
| | *E.cloacae* GN7471 | 0.063 | 8 | 0.031 | 0.031 | 0.5 |
| | *C.freundii* GN346 | 0.063 | 8 | 0.031 | 0.031 | 0.5 |
| | *P.aeruginosa* IFO3451 | 0.5 | 8 | 0.5 | 0.5 | 1.1 |
| | *P.aeruginosa* TL-2666 | 1 | 16 | 1 | 1 | 1.1 |
| | *P.aeruginosa* TL-2667 | 4 | 16 | 4 | 4 | 1.3 |
| | *S.maltophilia* IID1275 | >32 | >128 | >32 | >32 | N.C. |
| | *M.catarrhalis* ATCC25238 | ≦0.016 | ≦0.008 | ≦0.016 | ≦0.016 | N.C. |
| | *M.catarrhalis* ATCC8176 | ≦0.016 | ≦0.008 | ≦0.016 | ≦0.016 | N.C. |

Table 1-c

| | Strain No. | MEPM alone | SM alone | Concomitant (MEPM:SM=1:2) | | FIC index |
|---|---|---|---|---|---|---|
| | | | | MEPM | SM | |
| Gram positive bacteria | *S.aureus* 209P | 0.063 | ≦0.008 | ≦0.016 | ≦0.031 | N.C. |
| | *S.aureus* MS94088 (hetero-MRSA) | 4 | 0.25 | 0.125 | 0.25 | 1.03 |
| | *S.aureus* SP-7928 (homo-MRSA) | 32 | 1 | 1 | 2 | 2.0 |
| | *S.epidermidis* IAM1296 | 1 | 0.016 | ≦0.016 | ≦0.031 | ≦2.0 |
| | *M.luteus* ATCC9341 | 0.125 | ≦0.008 | ≦0.016 | ≦0.031 | N.C. |
| | *Str.pyogenes* Cook | ≦0.016 | ≦0.008 | ≦0.016 | ≦0.031 | N.C. |
| | *E.faecalis* ATCC19433 | 4 | 1 | 0.5 | 1 | 1.1 |
| | *E.faecium* ATCC19434 | 8 | 0.25 | 0.125 | 0.25 | 1.02 |
| | *B.subtilis* ATCC6633 | 0.063 | ≦0.008 | ≦0.016 | ≦0.031 | N.C. |
| Gram negative bacteria | *E.coli* NIHJ JC-2 | ≦0.016 | 0.125 | ≦0.016 | ≦0.031 | N.C. |
| | *E.coli* ML1410 | 0.031 | 0.25 | ≦0.016 | ≦0.031 | ≦0.6 |
| | *E.coli* ML1410 RP4 | 0.031 | 0.5 | ≦0.016 | ≦0.031 | ≦0.6 |
| | *K.pneumoniae* ATCC10031 | ≦0.016 | 0.031 | ≦0.016 | ≦0.031 | N.C. |
| | *P.mirabiris* GN2425 | 0.063 | 0.125 | 0.031 | 0.063 | 1 |
| | *P.vurgalis* OX-19 | 0.063 | 0.063 | ≦0.016 | ≦0.031 | ≦0.8 |
| | *P.vulgaris* GN7919 | 0.063 | 1 | 0.031 | 0.063 | 0.6 |
| | *S.marcescens* χ 100 | 0.031 | 0.5 | ≦0.016 | ≦0.031 | ≦0.6 |
| | *S.marcescens* GN6473 | 0.063 | 4 | 0.031 | 0.063 | 0.5 |
| | *E.aerogenes* ATCC13048 | 0.063 | 8 | 0.031 | 0.063 | 0.5 |
| | *E.cloacae* GN7471 | 0.063 | 8 | 0.031 | 0.063 | 0.5 |
| | *C.freundii* GN346 | 0.063 | 8 | 0.031 | 0.063 | 0.5 |
| | *P.aeruginosa* IFO3451 | 0.5 | 8 | 0.5 | 1 | 1.1 |
| | *P.aeruginosa* TL-2666 | 1 | 16 | 1 | 2 | 1.1 |
| | *P.aeruginosa* TL-2667 | 4 | 16 | 2 | 4 | 0.8 |
| | *S.maltophilia* IID1275 | >32 | >128 | >32 | >64 | N.C. |
| | *M.catarrhalis* ATCC25238 | ≦0.016 | ≦0.008 | ≦0.016 | ≦0.031 | N.C. |
| | *M.catarrhalis* ATCC8176 | ≦0.016 | ≦0.008 | ≦0.016 | ≦0.031 | N.C. |

Table 1-d

| | Strain No. | MEPM alone | SM alone | Concomitant (MEPM:SM=1:4) | | FIC index |
|---|---|---|---|---|---|---|
| | | | | MEPM | SM | |
| Gram positive bacteria | *S.aureus* 209P | 0.063 | ≦0.008 | ≦0.016 | ≦0.063 | N.C. |
| | *S.aureus* MS94088 (hetero-MRSA) | 4 | 0.25 | 0.063 | 0.25 | 1.02 |
| | *S.aureus* SP-7928 (homo-MRSA) | 32 | 1 | 0.5 | 2 | 2.0 |
| | *S.epidermidis* IAM1296 | 1 | 0.016 | ≦0.016 | ≦0.063 | ≦4.0 |
| | *M.luteus* ATCC9341 | 0.125 | ≦0.008 | ≦0.016 | ≦0.063 | N.C. |
| | *Str.pyogenes* Cook | ≦0.016 | ≦0.008 | ≦0.016 | ≦0.063 | N.C. |
| | *E.faecalis* ATCC19433 | 4 | 1 | 0.25 | 1 | 1.1 |
| | *E.faecium* ATCC19434 | 8 | 0.25 | 0.031 | 0.125 | 0.5 |
| | *B.subtilis* ATCC6633 | 0.063 | ≦0.008 | ≦0.016 | ≦0.063 | N.C. |
| Gram negative bacteria | *E.coli* NIHJ JC-2 | ≦0.016 | 0.125 | ≦0.016 | ≦0.063 | N.C. |
| | *E.coli* ML1410 | 0.031 | 0.25 | ≦0.016 | ≦0.063 | ≦0.8 |
| | *E.coli* ML1410 RP4 | 0.031 | 0.5 | ≦0.016 | ≦0.063 | ≦0.6 |
| | *K.pneumoniae* ATCC10031 | ≦0.016 | 0.031 | ≦0.016 | ≦0.063 | N.C. |
| | *P.mirabiris* GN2425 | 0.063 | 0.125 | ≦0.016 | ≦0.063 | ≦0.8 |
| | *P.vurgalis* OX-19 | 0.063 | 0.063 | ≦0.016 | ≦0.063 | ≦1.3 |
| | *P.vulgaris* GN7919 | 0.063 | 1 | 0.031 | 0.125 | 0.6 |
| | *S.marcescens* χ 100 | 0.031 | 0.5 | ≦0.016 | ≦0.063 | ≦0.6 |
| | *S.marcescens* GN6473 | 0.063 | 4 | 0.031 | 0.125 | 0.5 |
| | *E.aerogenes* ATCC13048 | 0.063 | 8 | 0.031 | 0.125 | 0.5 |
| | *E.cloacae* GN7471 | 0.063 | 8 | 0.031 | 0.125 | 0.5 |
| | *C.freundii* GN346 | 0.063 | 8 | 0.031 | 0.125 | 0.5 |
| | *P.aeruginosa* IFO3451 | 0.5 | 8 | 0.5 | 2 | 1.3 |
| | *P.aeruginosa* TL-2666 | 1 | 16 | 1 | 4 | 1.3 |
| | *P.aeruginosa* TL-2667 | 4 | 16 | 2 | 8 | 1 |
| | *S.maltophilia* IID1275 | >32 | >128 | >32 | >128 | N.C. |
| | *M.catarrhalis* ATCC25238 | ≦0.016 | ≦0.008 | ≦0.016 | ≦0.063 | N.C. |
| | *M.catarrhalis* ATCC8176 | ≦0.016 | ≦0.008 | ≦0.016 | ≦0.063 | N.C. |

Table 2-a

| | Strain No. | MEPM alone | SM alone | Concomitant (ME:SM=2:1) | | Concomitant (ME:SM=1:1) | | Concomitant (ME:SM=1:2) | | Concomitant (ME:SM=1:4) | |
|---|---|---|---|---|---|---|---|---|---|---|---|
| | | | | MEPM | SM | MEPM | SM | MEPM | SM | MEPM | SM |
| *Acinetobacter spp.* | 14712 | 1 | 2 | 0.5 | 0.25 | 0.5 | 0.5 | 0.5 | 1 | 0.25 | 1 |
| | Jan-54 | 1 | 2 | 0.5 | 0.25 | 0.5 | 0.5 | 0.5 | 1 | 0.25 | 1 |
| | Jan-99 | 0.25 | 0.5 | 0.25 | 0.125 | 0.25 | 0.25 | 0.125 | 0.25 | 0.125 | 0.5 |
| | 1399-2 | 0.5 | 2 | 0.5 | 0.25 | 0.5 | 0.5 | 0.5 | 1 | 0.25 | 1 |
| | 1398-2 | 32 | 8 | 4 | 2 | 2 | 2 | 2 | 4 | 1 | 4 |
| | 1195 | 0.25 | 0.5 | 0.25 | 0.125 | 0.25 | 0.25 | 0.125 | 0.25 | 0.063 | 0.25 |
| | 256 | 1 | 1 | 0.5 | 0.25 | 0.5 | 0.5 | 0.5 | 1 | 0.25 | 1 |
| | 227-1 | 1 | 2 | 0.5 | 0.25 | 0.5 | 0.5 | 0.5 | 1 | 0.25 | 1 |
| | Jan-81 | 4 | 32 | 4 | 2 | 8 | 8 | 2 | 4 | 2 | 8 |
| | Jan-99 | 0.5 | 2 | 0.5 | 0.25 | 0.5 | 0.5 | 0.25 | 0.5 | 0.125 | 0.5 |
| | 1338-2 | 1 | 0.5 | 0.5 | 0.25 | 0.5 | 0.5 | 0.5 | 1 | 0.125 | 0.5 |
| | 1198 | 0.5 | 0.5 | 0.25 | 0.125 | 0.25 | 0.25 | 0.125 | 0.25 | 0.063 | 0.25 |
| | 175 | 0.125 | 0.5 | 0.125 | 0.063 | 0.125 | 0.125 | 0.063 | 0.125 | 0.063 | 0.25 |
| | Concomitant effect | - | - | 7/13 (Strain) | | 7/13 (Strain) | | 12/13 (Strain) | | 13/13 (Strain) | |
| *S.marcescens* | 14424 | 4 | 16 | 4 | 2 | 8 | 8 | 2 | 4 | 2 | 8 |
| | 14689 | 4 | 32 | 4 | 2 | 2 | 2 | 2 | 4 | 2 | 8 |
| | 14425 | 0.031 | 2 | 0.031 | 0.016 | 0.031 | 0.031 | 0.031 | 0.063 | 0.031 | 0.125 |
| | 14991 | 1 | 32 | 1 | 0.5 | 2 | 2 | 1 | 2 | 1 | 4 |
| | 14981 | 1 | 8 | 1 | 0.5 | 1 | 1 | 0.5 | 1 | 0.5 | 2 |
| | 2054522 | 1 | 8 | 1 | 0.5 | 1 | 1 | 0.5 | 1 | 0.5 | 2 |
| | 2012884 | 4 | 8 | 4 | 2 | 2 | 2 | 2 | 4 | 1 | 4 |
| | 1944950 | 8 | 32 | 8 | 4 | 8 | 8 | 4 | 8 | 4 | 16 |
| | 1928513 | 1 | 8 | 1 | 0.5 | 1 | 1 | 0.5 | 1 | 0.5 | 2 |
| | 7155 | 2 | 8 | 2 | 1 | 2 | 2 | 1 | 2 | 0.5 | 2 |
| | 7138 | 2 | 8 | 2 | 1 | 2 | 2 | 1 | 2 | 0.5 | 2 |
| | Concomitant effect | - | - | 0/11 (Strain) | | 2/11 (Strain) | | 9/11 (Strain) | | 9/11 (Strain) | |

Table 2-b

| | Strain No. | MEPM alone | SM alone | Concomitant (ME:SM=2:1) | | Concomitant (ME:SM=1:1) | | Concomitant (ME:SM=1:2) | | Concomitant (ME:SM=1:4) | |
|---|---|---|---|---|---|---|---|---|---|---|---|
| | | | | MEPM | SM | MEPM | SM | MEPM | SM | MEPM | SM |
| *B.cepacia* | 14300 | 0.5 | 2 | 0.5 | 0.25 | 0.5 | 0.5 | 0.5 | 1 | 0.25 | 1 |
| | 14427 | 1 | 8 | 1 | 0.5 | 1 | 1 | 0.5 | 1 | 0.5 | 2 |
| | 14428 | 2 | 8 | 1 | 0.5 | 1 | 1 | 1 | 2 | 0.5 | 2 |
| | 14429 | 1 | 4 | 1 | 0.5 | 1 | 1 | 0.5 | 1 | 0.5 | 2 |
| | 14430 | 2 | 8 | 2 | 1 | 1 | 1 | 1 | 2 | 2 | 8 |
| | 14607 | 1 | 4 | 1 | 0.5 | 1 | 1 | 0.5 | 1 | 0.5 | 2 |
| | 7438 | 2 | 16 | 2 | 1 | 2 | 2 | 1 | 2 | 2 | 8 |
| | 1892324 | 2 | 4 | 2 | 1 | 1 | 1 | 1 | 2 | 0.5 | 2 |
| | 3138 | 2 | 8 | 2 | 1 | 1 | 1 | 1 | 2 | 2 | 8 |
| | 7247 | 2 | 16 | 2 | 1 | 2 | 2 | 2 | 4 | 2 | 8 |
| | 1993527 | 2 | 8 | 2 | 1 | 1 | 1 | 1 | 2 | 2 | 8 |
| | 257-1 | 0.5 | 4 | 0.5 | 0.25 | 0.5 | 0.5 | 0.25 | 0.5 | 0.25 | 1 |
| | Concomitant effect | - | - | 1/12(strain) | | 5/12(strain) | | 10/12(strain) | | 7/12(strain) | |
| *P.aeruginosa* | 13221 | 0.125 | 16 | 0.063 | 0.031 | 0.125 | 0.125 | 0.125 | 0.25 | 0.063 | 0.25 |
| | 13224 | 4 | 16 | 4 | 2 | 4 | 4 | 2 | 4 | 2 | 8 |
| | 13245 | 2 | 16 | 2 | 1 | 2 | 2 | 1 | 2 | 2 | 8 |
| | 13250 | 0.5 | 8 | 0.5 | 0.25 | 0.5 | 0.5 | 0.25 | 0.5 | 0.5 | 2 |
| | 13269 | 4 | 16 | 4 | 2 | 4 | 4 | 2 | 4 | 2 | 8 |
| | 13272 | 4 | 16 | 4 | 2 | 4 | 4 | 2 | 4 | 2 | 8 |
| | 13280 | 8 | 32 | 8 | 4 | 4 | 4 | 4 | 8 | 4 | 16 |
| | 13285 | 4 | 32 | 4 | 2 | 4 | 4 | 4 | 8 | 4 | 16 |
| | 12789 | 0.5 | 8 | 0.5 | 0.25 | 0.5 | 0.5 | 0.5 | 1 | 0.5 | 2 |
| | 12790 | 0.063 | 4 | 0.031 | 0.016 | 0.063 | 0.063 | 0.125 | 0.25 | 0.063 | 0.25 |
| | 15019 | 4 | 16 | 4 | 2 | 4 | 4 | 4 | 8 | 2 | 8 |
| | 15032 | 2 | 8 | 2 | 1 | 2 | 2 | 1 | 2 | 2 | 8 |
| | 15036 | 2 | 8 | 2 | 1 | 2 | 2 | 1 | 2 | 2 | 8 |
| | 15039 | 8 | 32 | 8 | 4 | 8 | 8 | 8 | 16 | 4 | 16 |
| | 15040 | 4 | 16 | 4 | 2 | 4 | 4 | 2 | 4 | 2 | 8 |
| | Concomitant effect | - | - | 2/15(strain) | | 1/15(strain) | | 9/15(strain) | | 8/15(strain) | |

Table 3-a

| | Strain No. | IPM alone | SM alone | Concomitant (IPM:SM=1:1) | | FIC index |
|---|---|---|---|---|---|---|
| | | | | IPM | SM | |
| Gram positive bacteria | *S.aureus* 209P | 0.008 | 0.008 | 0.004 | 0.004 | 1 |
| | *S.aureus* MS94088 (hetero-MRSA) | 0.125 | 0.25 | 0.25 | 0.25 | 3.0 |
| | *S.aureus* SP-7928 (homo-MRSA) | >32 | 2 | 2 | 2 | <1.1 |
| | *S.epidermidis* IAM1296 | 0.25 | 0.031 | 0.016 | 0.016 | 0.6 |
| | *M.luteus* ATCC9341 | 0.024 | 0.004 | 0.004 | 0.004 | 1.2 |
| | *Str.pyogenes* Cook | ≦0.002 | 0.004 | ≦0.002 | ≦0.002 | N.C. |
| | *E.faecalis* ATCC19433 | 1 | 2 | 0.5 | 0.5 | 0.8 |
| | *E.faecium* ATCC19434 | 4 | 0.25 | 0.125 | 0.125 | 0.5 |
| | *B.subtilis* ATCC6633 | 0.016 | 0.004 | ≦0.002 | ≦0.002 | ≦0.6 |
| Gram negative bacteria | *E.coli* NIHJ JC-2 | 0.125 | 0.125 | 0.063 | 0.063 | 1 |
| | *E.coli* ML1410 | 0.25 | 0.25 | 0.125 | 0.125 | 1 |
| | *E.coli* ML1410 RP4 | 0.5 | 0.5 | 0.125 | 0.125 | 0.5 |
| | *K.pneumoniae* ATCC10031 | 0.125 | 0.063 | 0.031 | 0.031 | 0.7 |
| | *P.mirabiris* GN2425 | 1 | 0.125 | 0.25 | 0.25 | 2.3 |
| | *P.vurgalis* OX-19 | 1 | 0.063 | 0.063 | 0.063 | 1.1 |
| | *P.vulgaris* GN7919 | 1 | 1 | 0.5 | 0.5 | 1 |
| | *S.marcescens* χ 100 | 0.25 | 1 | 0.125 | 0.125 | 0.6 |
| | *S.marcescens* GN6473 | 0.25 | 4 | 0.25 | 0.25 | 1.1 |
| | *E.aerogenes* ATCC13048 | 0.25 | 8 | 0.125 | 0.125 | 0.5 |
| | *E.cloacae* GN7471 | 0.125 | 8 | 0.125 | 0.125 | 1.02 |
| | *C.freundii* GN346 | 0.5 | 8 | 0.25 | 0.25 | 0.5 |
| | *P.aeruginosa* IFO3451 | 1 | 8 | 0.5 | 0.5 | 0.6 |
| | *P.aeruginosa* TL-2666 | 2 | 16 | 1 | 1 | 0.6 |
| | *P.aeruginosa* TL-2667 | 8 | 16 | 8 | 8 | 1.5 |
| | *S.maltophilia* IID1275 | >32 | >32 | >32 | >32 | N.C. |
| | *M.catarrhalis* ATCC25238 | 0.008 | ≦0.002 | ≦0.002 | ≦0.002 | N.C. |
| | *M.catarrhalis* ATCC8176 | 0.008 | ≦0.002 | ≦0.002 | ≦0.002 | N.C. |

Table 3-b

| Strain No. | | PAPM alone | SM alone | Concomitant (PAPM:SM=1:1) | | FIC index |
|---|---|---|---|---|---|---|
| | | | | PAPM | SM | |
| Gram positive bacteria | *S.aureus* 209P | 0.031 | 0.008 | 0.004 | 0.004 | 0.6 |
| | *S.aureus* MS94088 (hetero-MRSA) | 0.5 | 0.25 | 0.25 | 0.25 | 1.5 |
| | *S.aureus* SP-7928 (homo-MRSA) | >32 | 2 | 2 | 2 | <1.1 |
| | *S.epidermidis* IAM1296 | 0.5 | 0.031 | 0.016 | 0.016 | 0.5 |
| | *M.luteus* ATCC9341 | 0.031 | 0.004 | ≦0.002 | ≦0.002 | ≦0.6 |
| | *Str.pyogenes* Cook | 0.008 | 0.004 | ≦0.002 | ≦0.002 | ≦0.8 |
| | *E.faecalis* ATCC19433 | 2 | 2 | 1 | 1 | 1 |
| | *E.faecium* ATCC19434 | 8 | 0.25 | 0.125 | 0.125 | 0.5 |
| | *B.subtilis* ATCC6633 | 0.016 | 0.004 | ≦0.002 | ≦0.002 | ≦0.6 |
| Gram negative bacteria | *E.coli* NIHJ JC-2 | 0.25 | 0.125 | 0.063 | 0.063 | 0.8 |
| | *E.coli* ML1410 | 0.5 | 0.25 | 0.125 | 0.125 | 0.8 |
| | *E.coli* ML1410 RP4 | 0.5 | 0.5 | 0.125 | 0.125 | 0.5 |
| | *K.pneumoniae* ATCC10031 | 0.125 | 0.063 | 0.016 | 0.016 | 0.4 |
| | *P.mirabiris* GN2425 | 1 | 0.125 | 0.125 | 0.125 | 1.1 |
| | *P.vurgalis* OX-19 | 1 | 0.063 | 0.063 | 0.063 | 1.1 |
| | *P.vulgaris* GN7919 | 1 | 1 | 0.5 | 0.5 | 1 |
| | *S.marcescens* χ100 | 0.25 | 1 | 0.125 | 0.125 | 0.6 |
| | *S.marcescens* GN6473 | 0.5 | 4 | 0.25 | 0.25 | 0.6 |
| | *E.aerogenes* ATCC13048 | 0.5 | 8 | 0.25 | 0.25 | 0.5 |
| | *E.cloacae* GN7471 | 0.25 | 8 | 0.125 | 0.125 | 0.5 |
| | *C.freundii* GN346 | 0.5 | 8 | 0.25 | 0.25 | 0.5 |
| | *P.aeruginosa* IFO3451 | 4 | 8 | 2 | 2 | 0.8 |
| | *P.aeruginosa* TL-2666 | 8 | 16 | 8 | 8 | 1.5 |
| | *P.aeruginosa* TL-2667 | 16 | 16 | 16 | 16 | 2 |
| | *S.maltophilia* IID1275 | >32 | >32 | >32 | >32 | N.C. |
| | *M.catarrhalis* ATCC25238 | 0.008 | ≦0.002 | ≦0.002 | ≦0.002 | N.C. |
| | *M.catarrhalis* ATCC8176 | 0.008 | ≦0.002 | ≦0.002 | ≦0.002 | N.C. |

Table 3-c

| | Strain No. | BIPM alone | SM alone | Concomitant (BIPM:SM=1:1) | | FIC index |
|---|---|---|---|---|---|---|
| | | | | BIPM | SM | |
| Gram positive bacteria | *S.aureus* 209P | 0.031 | 0.004 | 0.008 | 0.008 | 2.3 |
| | *S.aureus* MS94088 (hetero-MRSA) | 4 | 0.25 | 0.25 | 0.25 | 1.1 |
| | *S.aureus* SP-7928 (homo-MRSA) | >32 | 1 | 2 | 2 | <2.1 |
| | *S.epidermidis* IAM1296 | 1 | 0.016 | 0.031 | 0.031 | 2.0 |
| | *M.luteus* ATCC9341 | 0.125 | ≦0.002 | 0.004 | 0.004 | N.C. |
| | *Str.pyogenes* Cook | 0.008 | ≦0.002 | 0.004 | 0.004 | N.C. |
| | *E.faecalis* ATCC19433 | 4 | 1 | 1 | 1 | 1.3 |
| | *E.faecium* ATCC19434 | 16 | 0.125 | 0.25 | 0.25 | 2.0 |
| | *B.subtilis* ATCC6633 | 0.031 | ≦0.002 | ≦0.002 | ≦0.002 | N.C. |
| Gram negative bacteria | *E.coli* NIHJ JC-2 | 0.031 | 0.125 | 0.031 | 0.031 | 1.2 |
| | *E.coli* ML1410 | 0.25 | 0.25 | 0.063 | 0.063 | 0.5 |
| | *E.coli* ML1410 RP4 | 0.25 | 0.5 | 0.125 | 0.125 | 0.8 |
| | *K.pneumoniae* ATCC10031 | 0.031 | 0.031 | 0.016 | 0.016 | 1.03 |
| | *P.mirabiris* GN2425 | 0.25 | 0.125 | 0.125 | 0.125 | 1.5 |
| | *P.vurgalis* OX-19 | 0.25 | 0.031 | 0.031 | 0.031 | 1.1 |
| | *P.vulgaris* GN7919 | 0.5 | 1 | 0.25 | 0.25 | 0.8 |
| | *S.marcescens* χ 100 | 0.063 | 1 | 0.063 | 0.063 | 1.1 |
| | *S.marcescens* GN6473 | 0.125 | 4 | 0.125 | 0.125 | 1.03 |
| | *E.aerogenes* ATCC13048 | 0.125 | 8 | 0.063 | 0.063 | 0.5 |
| | *E.cloacae* GN7471 | 0.063 | 8 | 0.063 | 0.063 | 1.01 |
| | *C.freundii* GN346 | 0.25 | 8 | 0.125 | 0.125 | 0.5 |
| | *P.aeruginosa* IFO3451 | 0.25 | 8 | 0.25 | 0.25 | 1.03 |
| | *P.aeruginosa* TL-2666 | 1 | 16 | 1 | 1 | 1.1 |
| | *P.aeruginosa* TL-2667 | 8 | 16 | 4 | 4 | 0.8 |
| | *S.maltophilia* IID1275 | >32 | >32 | >32 | >32 | N.C. |
| | *M.catarrhalis* ATCC25238 | 0.016 | ≦0.002 | ≦0.002 | ≦0.002 | N.C. |
| | *M.catarrhalis* ATCC8176 | 0.031 | ≦0.002 | ≦0.002 | ≦0.002 | N.C. |

Table 3-d

| | Strain No. | DRPM alone | SM alone | Concomitant (DRPM:SM=1:1) | | FIC index |
|---|---|---|---|---|---|---|
| | | | | DRPM | SM | |
| Gram positive bacteria | *S.aureus* 209P | 0.016 | 0.004 | 0.004 | 0.004 | 1.3 |
| | *S.aureus* MS94088 (hetero-MRSA) | 2 | 0.25 | 0.25 | 0.25 | 1.1 |
| | *S.aureus* SP-7928 (homo-MRSA) | 32 | 1 | 1 | 1 | 1.03 |
| | *S.epidermidis* IAM1296 | 0.5 | 0.016 | 0.031 | 0.031 | 2.1 |
| | *M.luteus* ATCC9341 | 0.031 | ≦0.002 | ≦0.002 | ≦0.002 | N.C. |
| | *Str.pyogenes* Cook | ≦0.002 | ≦0.002 | ≦0.002 | ≦0.002 | N.C. |
| | *E.faecalis* ATCC19433 | 4 | 1 | 1 | 1 | 1.3 |
| | *E.faecium* ATCC19434 | 8 | 0.125 | 0.25 | 0.25 | 2.0 |
| | *B.subtilis* ATCC6633 | 0.008 | ≦0.002 | ≦0.002 | ≦0.002 | N.C. |
| Gram negative bacteria | *E.coli* NIHJ JC-2 | 0.016 | 0.125 | 0.016 | 0.016 | 1.1 |
| | *E.coli* ML1410 | 0.031 | 0.25 | 0.031 | 0.031 | 1.1 |
| | *E.coli* ML1410 RP4 | 0.031 | 0.5 | 0.031 | 0.031 | 1.1 |
| | *K.pneumoniae* ATCC10031 | 0.016 | 0.031 | 0.008 | 0.008 | 0.8 |
| | *P.mirabiris* GN2425 | 0.063 | 0.125 | 0.063 | 0.063 | 1.5 |
| | *P.vurgalis* OX-19 | 0.031 | 0.031 | 0.031 | 0.031 | 2 |
| | *P.vulgaris* GN7919 | 0.125 | 1 | 0.063 | 0.063 | 0.6 |
| | *S.marcescens* χ100 | 0.031 | 1 | 0.031 | 0.031 | 1.03 |
| | *S.marcescens* GN6473 | 0.063 | 4 | 0.063 | 0.063 | 1.02 |
| | *E.aerogenes* ATCC13048 | 0.031 | 8 | 0.031 | 0.031 | 1.004 |
| | *E.cloacae* GN7471 | 0.031 | 8 | 0.031 | 0.031 | 1.004 |
| | *C.freundii* GN346 | 0.063 | 8 | 0.063 | 0.063 | 1.01 |
| | *P.aeruginosa* IFO3451 | 0.5 | 8 | 0.25 | 0.25 | 0.5 |
| | *P.aeruginosa* TL-2666 | 1 | 16 | 1 | 1 | 1.1 |
| | *P.aeruginosa* TL-2667 | 2 | 16 | 2 | 2 | 1.1 |
| | *S.maltophilia* IID1275 | >32 | >32 | >32 | >32 | N.C. |
| | *M.catarrhalis* ATCC25238 | 0.004 | ≦0.002 | ≦0.002 | ≦0.002 | N.C. |
| | *M.catarrhalis* ATCC8176 | 0.008 | ≦0.002 | ≦0.002 | ≦0.002 | N.C. |

Table 3-e

| | Strain No. | PIPC alone | SM alone | Concomitant (PIPC:SM=1:1) | | FIC index |
|---|---|---|---|---|---|---|
| | | | | PIPC | SM | |
| Gram positive bacteria | *S.aureus* 209P | 0.25 | 0.004 | 0.004 | 0.004 | 1.02 |
| | *S.aureus* MS94088 (hetero-MRSA) | >32 | 0.25 | 0.125 | 0.125 | <0.5 |
| | *S.aureus* SP-7928 (homo-MRSA) | >32 | 1 | 1 | 1 | <1.03 |
| | *S.epidermidis* IAM1296 | 2 | 0.016 | 0.016 | 0.016 | 1.01 |
| | *M.luteus* ATCC9341 | 0.031 | ≦0.002 | ≦0.002 | ≦0.002 | N.C. |
| | *Str.pyogenes* Cook | 0.031 | ≦0.002 | ≦0.002 | ≦0.002 | N.C. |
| | *E.faecalis* ATCC19433 | 2 | 1 | 0.5 | 0.5 | 0.8 |
| | *E.faecium* ATCC19434 | 8 | 0.125 | 0.125 | 0.125 | 1.02 |
| | *B.subtilis* ATCC6633 | 0.125 | ≦0.002 | ≦0.002 | ≦0.002 | N.C. |
| Gram negative bacteria | *E.coli* NIHJ JC-2 | 0.25 | 0.125 | 0.063 | 0.063 | 0.8 |
| | *E.coli* ML1410 | 2 | 0.25 | 0.125 | 0.125 | 0.6 |
| | *E.coli* ML1410 RP4 | >32 | 0.5 | 0.5 | 0.5 | <1.02 |
| | *K.pneumoniae* ATCC10031 | 0.25 | 0.031 | 0.016 | 0.016 | 0.6 |
| | *P.mirabiris* GN2425 | 0.25 | 0.125 | 0.063 | 0.063 | 0.8 |
| | *P.vurgalis* OX-19 | 0.008 | 0.031 | 0.008 | ≦0.002 | ≦1.1 |
| | *P.vulgaris* GN7919 | >32 | 1 | 0.5 | 0.5 | <0.5 |
| | *S.marcescens* χ100 | 0.5 | 1 | 0.25 | 0.25 | 0.8 |
| | *S.marcescens* GN6473 | >32 | 4 | 4 | 4 | <1.1 |
| | *E.aerogenes* ATCC13048 | 4 | 8 | 8 | 8 | 3.0 |
| | *E.cloacae* GN7471 | 16 | 8 | 8 | 8 | 1.5 |
| | *C.freundii* GN346 | 1 | 8 | 8 | 8 | 9.0 |
| | *P.aeruginosa* IFO3451 | 4 | 8 | 8 | 8 | 3.0 |
| | *P.aeruginosa* TL-2666 | 1 | 16 | 16 | 16 | 17 |
| | *P.aeruginosa* TL-2667 | 2 | 16 | 16 | 16 | 9.0 |
| | *S.maltophilia* IID1275 | >32 | >32 | >32 | >32 | N.C. |
| | *M.catarrhalis* ATCC25238 | 0.008 | ≦0.002 | ≦0.002 | ≦0.002 | N.C. |
| | *M.catarrhalis* ATCC8176 | 0.016 | ≦0.002 | ≦0.002 | ≦0.002 | N.C. |

Table 3-f

| Strain No. | | CAZ alone | SM alone | Concomitant (CAZ:SM=1:1) | | FIC index |
|---|---|---|---|---|---|---|
| | | | | CAZ | SM | |
| Gram positive bacteria | *S.aureus* 209P | 8 | 0.004 | 0.004 | 0.004 | 1.001 |
| | *S.aureus* MS94088 (hetero-MRSA) | >32 | 0.25 | 0.25 | 0.25 | <1.01 |
| | *S.aureus* SP-7928 (homo-MRSA) | >32 | 1 | 1 | 1 | <1.03 |
| | *S.epidermidis* IAM1296 | >32 | 0.016 | 0.031 | 0.031 | <2.0 |
| | *M.luteus* ATCC9341 | 1 | ≦0.002 | ≦0.002 | ≦0.002 | N.C. |
| | *Str.pyogenes* Cook | 0.125 | ≦0.002 | 0.004 | 0.004 | N.C. |
| | *E.faecalis* ATCC19433 | >32 | 1 | 1 | 1 | <1.03 |
| | *E.faecium* ATCC19434 | >32 | 0.125 | 0.125 | 0.125 | <1.004 |
| | *B.subtilis* ATCC6633 | 2 | ≦0.002 | ≦0.002 | ≦0.002 | N.C. |
| Gram negative bacteria | *E.coli* NIHJ JC-2 | 0.25 | 0.125 | 0.063 | 0.063 | 0.8 |
| | *E.coli* ML1410 | 0.25 | 0.25 | 0.063 | 0.063 | 0.5 |
| | *E.coli* ML1410 RP4 | 0.5 | 0.5 | 0.125 | 0.125 | 0.5 |
| | *K.pneumoniae* ATCC10031 | 0.031 | 0.031 | 0.008 | 0.008 | 0.5 |
| | *P.mirabiris* GN2425 | 0.063 | 0.125 | 0.031 | 0.031 | 0.7 |
| | *P.vurgalis* OX-19 | 0.031 | 0.031 | 0.008 | 0.008 | 0.5 |
| | *P.vulgaris* GN7919 | 0.5 | 1 | 0.5 | 0.5 | 1.5 |
| | *S.marcescens* χ100 | 0.25 | 1 | 0.125 | 0.125 | 0.6 |
| | *S.marcescens* GN6473 | 0.5 | 4 | 0.5 | 0.5 | 1.1 |
| | *E.aerogenes* ATCC13048 | 0.5 | 8 | 8 | 8 | 17 |
| | *E.cloacae* GN7471 | 32 | 8 | 8 | 8 | 1.3 |
| | *C.freundii* GN346 | 1 | 8 | 8 | 8 | 9.0 |
| | *P.aeruginosa* IFO3451 | 1 | 8 | 2 | 2 | 2.3 |
| | *P.aeruginosa* TL-2666 | 0.5 | 16 | 8 | 8 | 16.5 |
| | *P.aeruginosa* TL-2667 | 1 | 16 | 8 | 8 | 8.5 |
| | *S.maltophilia* IID1275 | >32 | >32 | >32 | >32 | N.C. |
| | *M.catarrhalis* ATCC25238 | 0.016 | ≦0.002 | ≦0.002 | ≦0.002 | N.C. |
| | *M.catarrhalis* ATCC8176 | 0.016 | ≦0.002 | ≦0.002 | ≦0.002 | N.C. |

Table 3-g

| | Strain No. | AZT alone | SM alone | Concomitant (AZT:SM=1:1) | | FIC index |
|---|---|---|---|---|---|---|
| | | | | AZT | SM | |
| Gram positive bacteria | *S.aureus* 209P | >32 | 0.004 | 0.008 | 0.008 | <2.0 |
| | *S.aureus* MS94088 (hetero-MRSA) | >32 | 0.25 | 0.25 | 0.25 | <1.008 |
| | *S.aureus* SP-7928 (homo-MRSA) | >32 | 1 | 2 | 2 | <2.1 |
| | *S.epidermidis* IAM1296 | >32 | 0.016 | 0.031 | 0.031 | <2.0 |
| | *M.luteus* ATCC9341 | 16 | ≦0.002 | ≦0.002 | ≦0.002 | N.C. |
| | *Str.pyogenes* Cook | 16 | ≦0.002 | 0.004 | 0.004 | N.C. |
| | *E.faecalis* ATCC19433 | >32 | 1 | 1 | 1 | <1.03 |
| | *E.faecium* ATCC19434 | >32 | 0.125 | 0.125 | 0.125 | <1.004 |
| | *B.subtilis* ATCC6633 | >32 | ≦0.002 | ≦0.002 | ≦0.002 | N.C. |
| Gram negative bacteria | *E.coli* NIHJ JC-2 | 0.063 | 0.125 | 0.063 | 0.063 | 1.5 |
| | *E.coli* ML1410 | 0.125 | 0.25 | 0.063 | 0.063 | 0.8 |
| | *E.coli* ML1410 RP4 | 0.125 | 0.5 | 0.063 | 0.063 | 0.6 |
| | *K.pneumoniae* ATCC10031 | 0.004 | 0.031 | 0.004 | 0.004 | 1.1 |
| | *P.mirabiris* GN2425 | 0.004 | 0.125 | 0.004 | 0.004 | 1.03 |
| | *P.vurgalis* OX-19 | 0.004 | 0.031 | 0.004 | 0.004 | 1.1 |
| | *P.vulgaris* GN7919 | 0.125 | 1 | 0.25 | 0.25 | 2.3 |
| | *S.marcescens* χ100 | 0.063 | 1 | 0.031 | 0.031 | 0.5 |
| | *S.marcescens* GN6473 | 0.125 | 4 | 0.25 | 0.25 | 2.1 |
| | *E.aerogenes* ATCC13048 | 0.063 | 8 | 8 | 8 | 128 |
| | *E.cloacae* GN7471 | 16 | 8 | 4 | 4 | 0.8 |
| | *C.freundii* GN346 | 0.5 | 8 | 8 | 8 | 17 |
| | *P.aeruginosa* IFO3451 | 2 | 8 | 2 | 2 | 1.3 |
| | *P.aeruginosa* TL-2666 | 2 | 16 | 8 | 8 | 4.5 |
| | *P.aeruginosa* TL-2667 | 2 | 16 | 8 | 8 | 4.5 |
| | *S.maltophilia* IID1275 | >32 | >32 | >32 | >32 | N.C. |
| | *M.catarrhalis* ATCC25238 | 0.125 | ≦0.002 | ≦0.002 | ≦0.002 | N.C. |
| | *M.catarrhalis* ATCC8176 | 0.25 | ≦0.002 | ≦0.002 | ≦0.002 | N.C. |

Table 3-h

| | Strain No. | CPFX alone | SM alone | Concomitant (CPFX:SM=1:1) | | FIC index |
|---|---|---|---|---|---|---|
| | | | | CPFX | SM | |
| Gram positive bacteria | *S.aureus* 209P | 0.063 | 0.004 | 0.008 | 0.008 | 2.1 |
| | *S.aureus* MS94088 (hetero-MRSA) | 0.125 | 0.25 | 0.125 | 0.125 | 1.5 |
| | *S.aureus* SP-7928 (homo-MRSA) | 0.25 | 1 | 0.25 | 0.25 | 1.3 |
| | *S.epidermidis* IAM1296 | 2 | 0.016 | 0.031 | 0.031 | 2.0 |
| | *M.luteus* ATCC9341 | 1 | ≦0.002 | 0.004 | 0.004 | N.C. |
| | *Str.pyogenes* Cook | 0.125 | ≦0.002 | 0.004 | 0.004 | N.C. |
| | *E.faecalis* ATCC19433 | 1 | 1 | 0.5 | 0.5 | 1 |
| | *E.faecium* ATCC19434 | 4 | 0.125 | 0.25 | 0.25 | 2.1 |
| | *B.subtilis* ATCC6633 | 0.008 | ≦0.002 | 0.004 | 0.004 | N.C. |
| Gram negative bacteria | *E.coli* NIHJ JC-2 | ≦0.002 | 0.125 | ≦0.002 | ≦0.002 | N.C. |
| | *E.coli* ML1410 | 0.125 | 0.25 | 0.125 | 0.125 | 1.5 |
| | *E.coli* ML1410 RP4 | 0.125 | 0.5 | 0.25 | 0.25 | 2.5 |
| | *K.pneumoniae* ATCC10031 | ≦0.002 | 0.031 | ≦0.002 | ≦0.002 | N.C. |
| | *P.mirabiris* GN2425 | 0.016 | 0.125 | 0.031 | 0.031 | 2.2 |
| | *P.vurgalis* OX-19 | 0.004 | 0.031 | 0.008 | 0.008 | 2.3 |
| | *P.vulgaris* GN7919 | 0.008 | 1 | 0.016 | 0.016 | 2.0 |
| | *S.marcescens* χ 100 | 0.063 | 1 | 0.063 | 0.063 | 1.1 |
| | *S.marcescens* GN6473 | 1 | 4 | 1 | 1 | 1.3 |
| | *E.aerogenes* ATCC13048 | 0.016 | 8 | 0.031 | 0.031 | 2.0 |
| | *E.cloacae* GN7471 | 0.016 | 8 | 0.016 | 0.016 | 1.002 |
| | *C.freundii* GN346 | 0.004 | 8 | 0.008 | 0.008 | 2.0 |
| | *P.aeruginosa* IFO3451 | 0.063 | 8 | 0.125 | 0.125 | 2.0 |
| | *P.aeruginosa* TL-2666 | 4 | 16 | 2 | 2 | 0.6 |
| | *P.aeruginosa* TL-2667 | 1 | 16 | 2 | 2 | 2.1 |
| | *S.maltophilia* IID1275 | 0.5 | >32 | 1 | 1 | <2.0 |
| | *M.catarrhalis* ATCC25238 | 0.031 | ≦0.002 | ≦0.002 | ≦0.002 | N.C. |
| | *M.catarrhalis* ATCC8176 | 0.016 | ≦0.002 | ≦0.002 | ≦0.002 | N.C. |

INDUSTRIAL APPLICABILITY

The concomitant medicament of the present invention shows the enhanced effect against various bacterial strains and is useful for prophylactic or therapeutic agent for various infectious diseases.

## Claims

1. An antimicrobial medicament containing a combination of a β-lactam compound represented by the following formula,

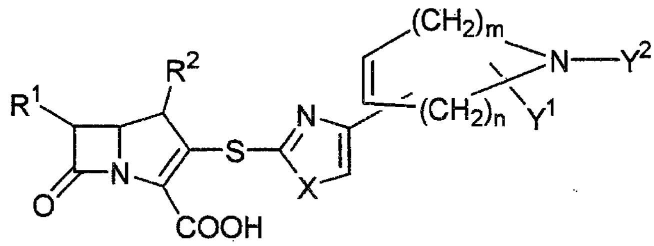

[1]

wherein $R^1$ is lower alkyl group or lower alkyl group substituted by hydroxy group, $R^2$ is hydrogen atom or lower alkyl group, X is O, S or NH, m and n are independently 0 to 4 and the sum of m and n is 0 to 4, $Y^1$ is halogen atom, cyano group, hydroxy group optionally protected, amino group optionally protected, lower alkyloxy group, lower alkylamino group, carboxyl group optionally protected, carbamoyl group optionally substituted or lower alkyl group optionally substituted, and $Y^2$ is hydrogen atom, lower alkyl group optionally substituted, cyano group or -C($R^3$)=N$R^4$ (wherein $R^3$ and $R^4$ are independently hydrogen atom, amino group optionally protected or substituted or lower alkyl group optionally substituted, or $R^3$ and $R^4$ may combine with the carbon atom and nitrogen atom to which they bond to form a 5 to 7 membered heterocyclic ring which may be substituted), provided that 1 to 4 $Y^1$s may present on the same ring and 2 $Y^1$s may present on the same carbon atom, or its pharmaceutically acceptable salt or its nontoxic ester; and a carbapenem.

2. The antimicrobial medicament according to claim 1, wherein in the formula [1] X is S, and the sum of m and n is 2 or 3.

3. The antimicrobial medicament according to claim 1 or 2, wherein in the formula [1] $R^1$ is 1-(R)-hydroxyethyl.

4. The antimicrobial medicament according to claim 1, wherein in the formula [1] $R^1$ is 1-(R)-hydroxyethyl, $R^2$ is methyl, X is S, the sum of m and n is 2 or 3, $Y^1$ is methyl or hydroxymethyl, and $Y^2$ is hydrogen atom.

5. The antimicrobial medicament according to claim 1, wherein the β-lactam compound [1] is (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid.

6. The antimicrobial medicament according to claim 1, wherein the β-lactam compound [1] is (4R,5S,6S)-6-[(1R)-1-hydroxyethyl]-3-({4-[(5R)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid, or (4R,5S,6S)-6-[(1R)-1-hydroxyethyl]-3-({4-[(5S)-5-(hydroxymethyl)-2,5-dihydro-1H-pyrrol-3-yl]-1,3-thiazol-2-yl}thio)-4-methyl-7-oxo-1-azabicyclo[3.2.0]hept-2-ene-2-carboxylic acid.

7. The antimicrobial medicament according to any one of claims 1 to 6, wherein the carbapenem is meropenem, imipenem, panipenem, biapenem, ertapenem, doripenem (S-4661), CS-023 or ME-1036.

8. An antimicrobial medicament containing combination of (4R, 5S,6S)-6-[(1R)-1-hydroxyethyl]-4-methyl-3-({4-[(5S)-5-methyl-2, 5-dihydro-1H-pyrrol-3-yl]-1,3-thiazol-2-yl}sulfanyl)-7-oxo-1-azabicyclo[3.2.0]hept-2-ene-2-carboxylic acid or its pharmaceutically acceptable salt; and meropenem, imipenem or panipenem.

9. An antimicrobial medicament containing combination of (4R, 5S, 6S)-6-[(1R)-1-hydroxyethyl]-4-methyl-3-({4-[(5S)-5-methyl-2, 5-dihydro-1H-pyrrol-3-yl]-1,3-thiazol-2-yl}sulfanyl)-7-oxo-1-azabicyclo[3.2.0]hept-2-ene-2-carboxylic acid, or its pharmaceutically acceptable salt; and meropenem.

**10.** The antimicrobial medicament according to any one of claims 1 to 9, wherein the β-lactam compound [1], its pharmaceutically acceptable salt or its nontoxic ester; and a carbapenem form a concomitant drug.

**11.** The antimicrobial medicament according to any one of claims 1 to 9, wherein the medicament is an injection.

**12.** The antimicrobial medicament for injection, wherein the injection contains (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}sulfanyl)-7-oxo-1-azabicyclo[3.2.0]hept-2-ene-2-carboxylic acid or its pharmaceutically acceptable salt; and meropenem as active ingredients.

**13.** The antimicrobial medicament according to claim 1 which is in the form of an injection or a vial or a kit for administration of the β-lactam compound or its pharmaceutically acceptable salt; and meropenem at same time or an interval.

**14.** A method for treating infectious diseases comprising administering combination of a β-lactam compound [1] or its pharmaceutically acceptable salt; and a carbapenem in the effective amount to a patient who needs it.

**15.** A method for treating infectious diseases comprising administering a concobinant comprising a β-lactam compound [1] or its pharmaceutically acceptable salt; and a carbapenem in the effective amount to a patient who needs it.

**16.** Use of combination of a β-lactam compound [1] or its pharmaceutically acceptable salt; and a carbapenem in the preparation of an antimicrobial medicament for treating infectious diseases.

**17.** Use of a β-lactam compound [1] or its pharmaceutically acceptable salt for combination of a carbapenem in the preparation of an antimicrobial medicament for treating infectious diseases.

**INTERNATIONAL SEARCH REPORT**

International application No.
PCT/JP2005/016611

A. CLASSIFICATION OF SUBJECT MATTER
***A61K31/427*** (2006.01), ***A61K31/407*** (2006.01), ***A61P31/04*** (2006.01), *C07D477/00* (2006.01)

According to International Patent Classification (IPC) or to both national classification and IPC

B. FIELDS SEARCHED

Minimum documentation searched (classification system followed by classification symbols
***A61K31/427*** (2006.01), ***A61K31/407*** (2006.01), ***A61P31/04*** (2006.01), *C07D477/00* (2006.01)

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | UEDA, Y. and SUNAGAWA, M., In Vitro and In Vivo Activities of Novel 2-(Thiazol-2-ylthio)-1 beta-Methylcarbapenems with Potent Activities against Multiresistant Gram-Positive Bacteria, Antimicrobial Agents and Chemotherapy, August 2003, Vol.47, No.8, pages 2471 to 2480 | 1-13,16,17 |
| Y | SUNAGAWA, M. et al., New Anti-MRSA and Anti-VRE Carbapenems: Synthesis and Structure-activity Relationships of 1 beta-Methyl-2-)thiazol-2-ylthio)carbapenems, The Journal of Antibiotics, August 2002, Vol.55, No.8, pages 722 to 757 | 1-13,16,17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 October, 2005 (06.10.05) | 25 October, 2005 (25.10.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2005/016611

C (Continuation). DOCUMENTS CONSIDERED TO BE RELEVANT

| Category* | Citation of document, with indication, where appropriate, of the relevant passages | Relevant to claim No. |
|---|---|---|
| Y | WO 02/38564 A1 (Sumitomo Pharmaceuticals Co., Ltd.),<br>16 May, 2002 (16.05.02),<br>Full text; particularly, pages 15 to 22, table 1; pages 143 to 146, Claims<br>& AU 200211006 A & EP 1340756 A1<br>& KR 2003048104 A & JP 2002-541097 A<br>& US 2004102433 A1 & CN 1484645 A | 1-13,16,17 |
| Y | JP 2002-525275 A (Merck & Co., Inc.),<br>13 August, 2002 (13.08.02),<br>Full text; particularly, page 1, lines 18 to 22; page 11, Claims 8 to 10; pages 16 to 17, Par. Nos. [0004] to [0005]; page 39, Par. No. [0069]; pages 42 to 45, Par. Nos. [0083] to [0089]; page 48, Fig. 1; page 51, Fig. 4<br>& WO 00/12048 A2 & AU 9961335 A<br>& EP 1115419 A2 | 1-13,16,17 |

Form PCT/ISA/210 (continuation of second sheet) (January 2004)

**INTERNATIONAL SEARCH REPORT**

International application No.
PCT/JP2005/016611

**Box No. II Observations where certain claims were found unsearchable (Continuation of item 2 of first sheet)**

This international search report has not been established in respect of certain claims under Article 17(2)(a) for the following reasons:

1. [X] Claims Nos.: 14, 15
   because they relate to subject matter not required to be searched by this Authority, namely:
   Claims 14 and 15 involve embodiments concerning methods for treatment of the human body by therapy and thus relate to a subject matter which this International Searching Authority is not required, under the provisions of Article 17(2)(a)(i) of the PCT (continued to extra sheet)

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

INTERNATIONAL SEARCH REPORT

International application No.
PCT/JP2005/016611

Continuation of Box No.II-1 of continuation of first sheet(2)

and Rule 39.1(iv) of the Regulations under the PCT, to search.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0238564 A
- JP 5294970 A
- WO 0242321 A


### Non-patent literature cited in the description


- *Journal of Antibiotics,* 1996, vol. 49 (2), 199-205
- *Journal of Antibiotics,* 2003, vol. 56 (6), 565-579
- *Chemotherapy,* 1981, vol. 129, 76-79
- *The Japanese Journal of Antibiotics,* 2005, vol. 58, 168-178